# EUROPEAN PATENT APPLICATION

(11) **EP 2 113 245 A2**
(43) Date of publication of application: **04.11.2009**
(21) Application number: 09007897.3
(22) Date of filing: 21.08.2002
(51) Int. Cl.: A61K 8/97, A61K 35/20, A61Q 19/08, A61K 36/48

(54) **Substances capable of potentiating laminin 5 productivity in epidermal cells and their use**

(30) Priority: 21.08.2001 JP 2001250285; 21.08.2001 JP 2001250289; 21.08.2001 JP 2001250295; 22.02.2002 JP 2002046014
(62) Divisional of application: 02794832.2
(71) Applicant: SHISEIDO COMPANY, LTD., Chuo-ku, Tokyo 104-8010 (JP)
(72) Inventor: Takada, Keiko, Yokohama-shi Kanagawa 224-8558 (JP); Amano, Satoshi, Yokohama-shi Kanagawa 236-8643 (JP); Nishiyama, Toshio, Yokohama-shi Kanagawa 236-8643 (JP)
(74) Representative: Albrecht, Thomas

(57) **Abstract**

The invention provides cosmetics or dermatological compositions comprising plant extract or whey extract having an action to potentiate laminin 5 productivity in the epidermal cells. They have mainly the action and efficacy to prevent or improve aging in the skin.

## Description

### Technical Field

This invention belongs to fields of cosmetics or dermatological technology. More specifically, the invention relates to compositions for external application to the skin, which contain substances capable of potentiating laminin 5 productivity in epidermal cells and of preventing skin aging by taking care of the basement membrane of human skin. The invention also relates to a method of preventing or improving skin aging with use of such compositions.

### Background Art

As drug for potentiating laminin 5 productivity in epidermal cells, soybean-derived preparations, lysophosphatidyl choline, lysophosphatidic acid and the like are known (JP 1999-343226A, JP 2000-226308A). When presence of suggestions on possible participation of versatile mechanisms for maintaining normal characteristics and functions of the skin is considered, it would be desirable to provide a substance which could belong to a category differing from said drug or preparations or could be derived from different origin and which is capable of potentiating productivity of laminin 5 in the epidermal cells.

### Disclosure of the Invention

The objects of the present invention are to provide a substance potentially containing a compound which may belong to a category differing from said lysophosphatidyl choline and the like, said substance being capable of potentiating productivity of said laminin 5; to provide anti-aging compositions for the skin, which contain said substance; and to provide a method for preventing or improving skin aging, which comprises topically administering said compositions to the skin of individuals.

With the view to accomplish the above objects, we have investigated laminin productivity promoting ability of large varieties of substances. In consequence, we have come to discover: of Ononis extract said to contain isoflavone glucoside, triterpenoid or tannin; Melilot extract said to contain coumarin, coumaric acid and the like; and *adzuki*-bean extract said to contain saponin and the like, which are known to exhibit anti-trichogenous action, anti-inflammation and anti-mutagenesis (cf. JP 1999-322548A, JP 1990-282332A and JP 2000-226332A, respectively), those which have undergone extraction operations unique for individual occasions are capable of potentiating productivity of said laminin 5 and in consequence are capable of preventing or improving deterioration in "moist feeling" or "tension" in the skin, which is said to be one cause of aging phenomena.

We have also discovered: of plant extracts selected from *Kakkon* extract, licorice extract, blackberry lily extract, extract of Alstonia scholaris, extract of Tinospora tuberculata Beumee and fenugreek extract which are known to exhibit, respectively, antioxidizing action (JP 1994-24937A), cell-activating action (JP 1985-23325A), cell-activating action (JP 1995-138179A), collagen production promoting action and anti-tyrosinase action (JP 1997-87164A), hyaluronic acid production promoting action and anti-tyrosinase action (JP 1998-29923A, JP 1997-30950A), and similarly hyaluronic acid production promoting action and anti-tyrosinase action (JP 1998-29924A, JP 1997-30950A), those which have undergone extraction operations unique for individual occasions could potentiate production of laminin 5 in the epidermal cells and could prevent or improve skin aging, similarly to said Onosis extract and the like. It has been also confirmed that whey extracts, apart from plant origin, also have similar activities.

There was also a report suggesting that *Kakkon* extract (an extract obtained from *kakkon* with 50% ethanol) "could be expected to serve as a material having skin wrinkle-ameliorating action" because the extract had an action to accelerate collagen synthesis by enderon fibroblasts (H. Mori, et al., Nippon Cosmetic technologists' Meeting, 2000, Osaka, Collection of Manuscripts, pp.25-27). This Collection of Manuscripts states the active component to accelerate synthesis of collagen is 4',7-DHIF(4',7-dihydroxyisoflavone).

There is furthermore a suggestion that isoflavone glucoside [e.g., 8-β-D-glucopyranosil-7-hydroxy-3-(3',4'-dihydroxyphenyl)-4H-1-benzopyran-4-onel which is contained in *Kakkon* extract would contribute to anti-aging of the skin, based on its action to eliminate active oxygen in an evaluation system using enzymes (e.g., JP 1992-282305A).

Whereas, we have discovered: extracts of plants which have never been used as active ingredient of medicines for external application to the skin so far as we are aware of, selected from plants belonging to Papaveraceae Bocconia, Legminosae Psophocarpus, Legminosae Cassia and Legminosae Erythraea Canchalagua (botanical name: Erythraea chilensis or Gentiana canchalagua), also have the action to potentiate productivity of laminin 5 in the epidermal cells and in consequence can prevent or improve aging in the skin.

According to the present invention, therefore, compositions for preventing or improving aging in the skin are provided, which comprises an effective amount of a substance capable of potentiating productivity of laminin 5 in the epidermal cells and components other than said substance, which can be customarily blended in cosmetics or medicines for external application to the skin.

Said substance used in the compositions is one or more kinds selected from the group consisting of plant extracts and whey extracts, excluding soybean seed extract.

The invention also provides the use of said substances for formulating the blended compositions for preventing or improving aging in the skin.

Furthermore, the invention provides a method of preventing or improving aging in the skin, said method comprising topical administration of an effective amount of the substance(s) onto the skin of an individual (e.g., human) who desires to prevent or improve aging in the skin (e.g., inhibition of wrinkle formation or reduction of wrinkles).

### Brief Explanation of Drawings

Fig. 1 is a graph showing the effect of later described (IV) winged bean extract to inhibit wrinkle formation by UVB, by comparing TEWL values.
Fig. 2 is a graph showing said inhibition effect by comparing skin thickness.
Fig. 3 is a graph showing said inhibition effect by wrinkle score following a table of visual rating standard of wrinkles.

### Best Mode for Carrying Ont the Invention

Laminin 5 is a main component of the structure called epidermal basement membrane which is located at the dermal-epidermal junction and composed of various glycoproteins and proteoglycans (Rouselle, et al., J. Cell Biol., 114, 567, 1991). Genetic deficiency of laminin 5 is known to induce epidermal hydroa, indicating that laminin 5 is an essential protein for binding epidermis to dermis (Aberdam, et al., Nat. Genet., 6, 299, 1994). Laminin 5 is also known to promote epidermal basement membrane formation (Tsunenaga, et al., Matrix Biology, 17, 603, 1998). While no theoretical restriction is intended, more specifically laminin 5 contributes to maintain or repair normal basement membrane structure or its functions and furthermore can retard progress in skin aging by repairing structural change in the basement membrane, which is caused by injury incurred by external stress such as ultraviolet rays and drying and internal stress such as mental stress occurring in daily life. In other words, potentiation of productivity of said laminin 5 is useful for maintenance of normal basement membrane structure of human skin, and in consequence is useful for prevention of, or improvement in, aging of the skin. We have confirmed that the anti-aging agents in accordance with the present invention potentiate production of laminin 5 in the epidermal cells and contribute to prevention of, or improvement in, skin aging. The term, anti-aging, as used herein signifies to prevent or improve deterioration in skin function caused by accumulation of structural change in basement membrane with aging or aging under photo radiation, more specifically wrinkles, flabbiness or hardening of the skin, whereby maintaining resilient, youthful and healthy skin condition.

"Ononis" said in this invention signifies the plant body generally referred to as Ononis root, comprising rhizomes and roots of Ononis spinosa L. (Legminosae) or of plants homologous thereto. Hence, Ononis extract refers to an extract obtainable by extraction of such plant body (available as crude drug in general) with lower alkanol such as 1,3-butylene glycol and ethanol. Whereas, said "Ononis extract" according to the invention encompasses extracts of plant bodies other than Ononis rhizomes and roots, which are obtained by other extraction methods, so long as such extracts meet the purpose of this invention.

"Melilot" signifies the whole of Melilot grass (western melilot) e.g., Melilotus afficinalis L (Legminosae) or plants homologous thereto, which are referred to as Melilot grass in the art of crude drug. Therefore, Melilot extract refers to the extracts obtainable by extraction of the whole of Melilot grass with lower alkanol such as ethanol, or lower alkylene glycol such as propylene glycol, 1,3-butylene glycol and the like. Whereas, "Melilot extract" of the present invention encompasses extracts from any parts, not the whole, of Melilot grass, so long as they meet the purpose of the invention.

"*Moyashi*" (vegetables artificially grown in the shade) signifies plant bodies obtained through artificial germination and growing of seeds of Legminosae plants such as soybean (Glycine max Merill) or Mung bean (Phaseolus radiatus L.), *adzuki* (Phaseolus ansularis Wight) and the like; or seeds of Black mappe, Japanese radish, alfalfa, buckwheat and the like. Accordingly, *Moyashi* extracts signify those obtained by extraction of said plants with various solvents.

*Adzuki* extract encompasses extracts of seeds of *adzuki* (Phaseolus angularis Wight) and plants homologous thereto, with water or with other solvents. As the preferred of such extracts, *adzuki* bulk meeting Japanese Cosmetic Ingredients Codex can be named.

As other plant extracts of the present invention, those obtained by using C₁-C₆ alkyl- C₁-C₆ alkylketone such as acetone, methyl ethyl ketone and the like; or C₂-C₆ alkanoic acid-C₁-C₆ alkyl such as ethyl acetate, butyl acetate and the like as the extraction solvent are preferred.

The term, "homologous plant", as used in this specification signifies those which are natural modification strains of exemplified seeds or strains or of plants closely related thereto, which contain similar active ingredients to those in the exemplified seeds or strains.

Of the extracts derived from *Kudzu* (Pueraria hirsuta Matsumura. or P. lobata Ohwi or P. thunbergiana Benth.), those derived from *Kakkon* [also referred to as Puerariae Radix] (storage *Kudzu* root finely shredded and dried: those commercially available as crude drugs led by officinal prescriptions can be used) are preferred. In general, such extracts obtained by extracting *Kakkon* with C₁-C₆ lower alkanol including substantially anhydrous methanol or ethanol are preferred.

Now, the isoflavones and isoflavone glucoside which are normally obtained by extracting *Kakkon* with an aqueous solvent such as hydrous ethanol, as described in earlier cited Collection of Manuscripts by Mori, et al. or JP 1992-282305A, [e.g., 4'-7-DHIF (or daidzein), and glucosides such as daidzin (daidzein-7-glucoside) and Puerarin (daidzein-8-glucoside)] show little or no laminin 5 productivity potentiating action as referred to in the present specification. Nevertheless, the fractions obtained by extracting *Kakkon* with substantially water-free methanol, ethanol or the like following the present invention as above possess laminin 5 productivity potentiating action, regardless whether they contain said isoflavones or isoflavone glucosides or not. Here "substantially water-free" signifies that the solvent contains no more than 10%, preferably no more than 5%, inter alia, no more than 3%, of water even when it contains water. Thus, those extracts according to the invention, in particular, *Kakkon* per se or extracts derived from *Kakkon*, are significant in that they contain, as the active components exhibiting laminin 5 productivity potentiating action, lupeol and isobauerenol of the following respective formulae, which exhibit higher oleophilicity than that of above-named glucosides.

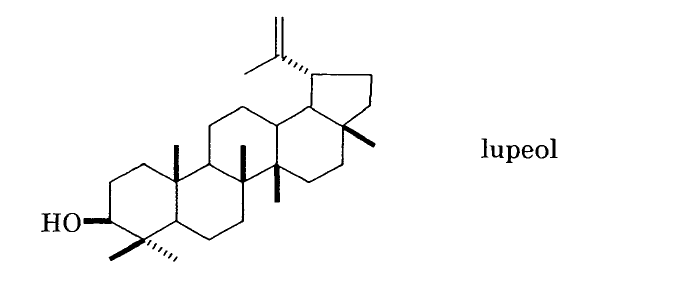

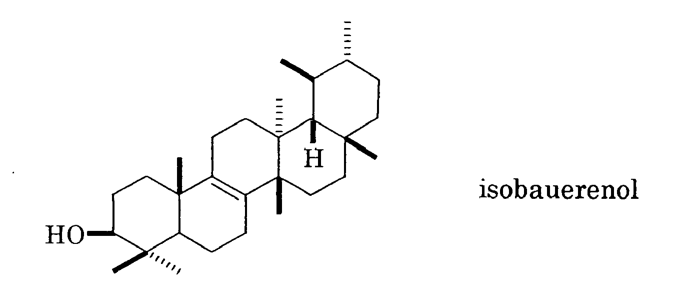

Accordingly, extracts derived from roots of *Kudzu* (Pueraria hirsuta Matsumura) or of plants homologous thereto (e.g., P. lobata Ohwi, P. thunbergiana Benth) following the present invention, in particular, *Kakkon* (or Pueraria Radix) per se or further fractions derived from *Kakkon*, for example, extracts obtained by extracting *Kakkon* with methanol or ethanol at room temperature; the fraction obtained by further distributing said extracts with a mixed solvent system of water and an organic solvent which is substantially immiscible with water (e.g., ethyl acetate) and recovering the fraction from the organic solvent phase; or the fraction obtained by extraction from said aqueous phase used for the distribution, with an organic solvent which is substantially immiscible with water (e.g., n-butanol), are generally particularly preferred, because said fractions are enriched with said lupeol and/or isobauerenol and highly suitable for the purpose of this invention. Obviously, purified lupeol and/or isobauerenol can be used as Kudzu-derived extract.

As licorice extract, licorice extract bulk meeting Japanese Cosmetic Ingredients Codex, liquid licorice extract or licorice flavonoid (oil-soluble licorice extract) are preferred. Obviously, those obtained by extracting roots of licorice (Glycyrrhiza glabra L. or Glycyrrhiza uralensis Fisher) or of plants homologous thereto and which meet the purpose of this invention are encompassed by the licorice extract as referred to in this invention. Besides those meeting said Japanese Cosmetic Ingredients Codex, officinal crude licorice extract and officinal licorice extract can also be conveniently used.

Blackberry lily extracts are obtained by extracting dry rhizomes of blackberry lily (Belamcanda chinensis DC. and of plants homologous thereto (available as crude drug *Yakan*) with various solvents.

All of Alstonia scholaris, Tinospora tuberculata Beumee, and fenugreek (Trigonella foenum-graecum) are native wild plants in, for example, Japan. Extracts from those or homologous plants thereto can be obtained by immersing or heating under reflux leaves, stems, branches, flowers, barks, seeds, fruits, rhizomes or whole of the plants in or with an extraction solvent, filtering the system and concentrating the filtrate. The extraction solvent used in that occasion may be any of those customarily used for extraction, examples of which including organic solvents such as earlier named alkanols (or alcohols), esters and glycols; or their mixtures with water where necessary. Typically, Alstonia scholaris extract is obtained by extracting bark of the plant with ethanol; Tinospora tuberculata Beumee extract, by extracting the whole of said plant with 1,3-butanediol, and fenugreek extract, by extracting its seeds with ethanol.

On the other hand, whey extract is formed of whey-derived components obtained by treating mammalian whey with acid and alcohol and removing insoluble matter. Preferably the mammalian whey is one obtained by filtering de-fatted mammalian milk through Celite™. The whey is preferably one or more kinds selected from those derived from milk of human, cow, goat, sheep and sow.

Generally the foregoing extracts are further diluted with ethanol or the like, or their dry products are allowed to retain the dry state or re-dissolved in, for example, ethanol, to serve as the active ingredient following the present invention.

As the starting materials for plant extracts which can be used in the present invention and deemed to have never been used in medicines for external application to the skin before, the following can be named.

As the typical of Papaveraceae Bocconia plants, Palo Amarillo, (botanical name: Bocconia arborea) natively growing in, for example, Mexico can be named. As the typical of Legminosae Psophocarpus plants, winged bean natively growing in, for example, Japan can be named. Retama (botanical name: Cassia spinecens H.) growing wild in Nepal is the typical of Legminosae Cassia plants; and Canchalagua (botanical name: Erythraea chilensis or Gentiana canchalagua) belonging to Legminosae Erythraea plants grows wild in Peru. Of these, winged bean is also called four angled bean or bird's-foot trefoil.

Extracts of above-named wild plants are obtained by immersing or heating under reflux leaves, stems, branches, flowers, barks, seeds, fruits, rhizomes or the whole of said plants in, or with, an extraction solvent, filtering the system and concentrating the filtrate. The extraction solvent may be any one of those customarily used for extraction, in particular, alcohols such as methanol, ethanol and the like; hydrous alcohols in certain occasions; and organic solvents such as acetone, ethyl acetate and the like. They may be used either singly or in combination. Thus obtained extracts may be blended in the compositions used according to the present invention, either as they are or further diluted with ethanol, or in dried state or their dried products may be re-dissolved in, for example, ethanol.

Concerning the extracts serving as the active ingredient according to the present invention, plants that can be their sources are exemplified in the foregoing. Whereas, all extracts derived from seeds or strains of plants closely related thereto (homologous plants) which exhibit equivalent or higher laminin 5 productivity in the epidermal cells to or than that of later described specific extracts are encompassed within the scope of the active ingredient of the present invention.

The amount of the foregoing plant extracts or whey extracts to be blended in the compositions or agents for external application to the skin according to the present invention cannot be specified because the optimum amount varies depending on the preparation forms, while it is broadly variable within a range not impairing the intended effect of the present invention. Individual amounts to be blended can be conveniently decided, by advance evaluation of laminin 5 productivity of each extract by the later described rating method, where necessary. The proviso, "where necessary" is added because the contents of the active ingredients in these extracts are not necessarily constant, depending on place and time of gathering their raw materials. Whereas, generally speaking, those plant extracts commercially available as crude drug including officinal prescriptions and those meeting Japanese Cosmetic Ingredients Codex often have approximately constant contents of the active ingredients.

The compositions or agents for external application to the skin according to the present invention may take any preparation forms suitable for external application to the skin or for topical administration to the skin. Normally, they can take such preparation forms as ointment, cream, milky lotion, lotion, pack, bath salt and the like.

Those composition or agents for external application according to the present invention may contain, depending on the selected preparation form and also where necessary, besides one or more of above-described essential components, additive components customarily used for cosmetics or medicines for external application to the skin, which are adequately selected from, for example, other laminin production accelerating agents, anti-aging agents, humectants, antioxidants, oleaginous components, UV absorbers, surfactants, thickeners, antiseptics, alcohols, pH regulators, detergents, desiccants, emulsifiers, powdery components, colorants, aqueous components, colorants, aqueous components, water, various skin tonics and perfumes. Methods for blending these can follow those known per se.

Besides the foregoing, sequestering agents such as disodium edetate, trisodium edetate, sodium citrate, sodium polyphosphate, sodium metaphosphate and gluconic acid; laminin 5 production promoting agents such as caffeine, tannin, verapamil, tranexamic acid and derivatives thereof, various crude drugs, tocopherol acetate, glycyrrhizic acid and derivatives or salts thereof, glycyrrhitinic acid derivatives, salicylic acid derivatives, lysophosphatidyl choline or lysophosphatidic acid and soybean preparations; sugars such as glucose, fructose, mannose, sucrose and trehalose; whitening agents such as arbutin and kojic acid; blood circulation promoters such as nonylic acid vanillylamide, benzyl nicotinate, β-butoxyethyl nicotinate, capsaicin, gingerone, cantharis tincture, ichthammol, caffeine, tannic acid, α-borneol, tocopherol nictinate, inositol hexanicotinate, cyclandelate, cinnarizine, tolazoline, acetylcholine, verapamil, cepharanthine and γ-oryzanoh antiseborrheic agents such as sulfur and thianthol, substances effective for various purposes, such as hinokitiol, zinc oxide, allantoin, turmeric extract, Bupleurum falcatum, Thymus quinguecostatus Celak, blackberry lily extract, catechu extract, Japanese beech sprout extract, hydrolyzed caseine, rice extract hydrolyzate, rice bran extract, Persicae Semen extract, Sophora angustifolia Sieb et Zucc. extract, thiotaurine, hypotaurine, Marjoram extract, silica-coated zinc oxide, Pyrola japonica Klenze extract, xylytol, arginine and its hydrochloride, serine, phellodedron bark extract components, coptidis rhizome extract components, lithospermum root extract components, peony root extract components, swertia herb extract components, birch extract components, sage extract components, loquat extract components, ginseng tract components, aloe extract components, mallow extract components, iris extract components, grape extract components, coix seed tract components, sponge gourd extract components, lily extract components, saffron extract components, cnidium rhizome extract components, zingiberis rhizome extract components, syoorengyo extract components, rosemary tract components, garlic extract components, capsicum extract components, burnet extract components, citrus unshiu peel and Japanese angelica root; vitamin A compounds such as retinol and retinol acetate; vitamin B₂ compounds such as riboflavin, riboflavin butyrate and flavin adenine dinucleotide; vitamin B₂ compounds such as pyridoxine hydrochloride and pyridoxine dioctanoate; vitamin C compounds such as L-ascorbic acid, L-ascorbic acid dipalmitate, L-ascorbic acid 2-sulfate sodium salt, L-ascorbic acid phosphate and DL-α-tocopherol-L-ascorbic acid diphosphate dipotassium salt; pantothenic acid compounds such as calcium pantothenate, D-pantothenyl alcohol, pantothenyl ethyl ether and acetylpantothenyl ethyl ether; vitamin D compounds such as ergocalciferol and cholecarciferol; nicotinic acid compounds such as nicotinic acid, nicotinic acid amide and benzyl nicotinate; vitamin E compounds such as α-tocopherol, tocopherol acetate, DL-α-tocopherol nicotinate and DL-α-tocopherol succinate; and other vitamins such as vitamin P and biotin, may be suitably incorporated.

Thus formulated compositions or drug for external application to the skin according to the present invention can be administered to the skin once or more times per day, because above-described active ingredients therein have substantially no detrimental effect on human skin, or they can be administered every day. Adequate administration schedules can be determined after simple application tests with cooperating volunteers.

The invention is explained referring to specific Examples hereunder, it being understood that the invention is in no way limited to the following Examples.

### (I) Preparation examples of plant extracts

Typical preparation examples of plant extracts which are not commercialized are shown in the following.

### (I-1) Palo Amarillo extract

Immersing 50 g of woody part of Palo Amarillo in ethanol at room temperature for a week and concentrating the resultant extract, 7.2 g of an ethanol extract was obtained.

### (I-2) Winged bean extract

Immersing 50 g of seed part of winged bean in ethanol at room temperature for a week and concentrating the resultant extract, 4.6 g of an ethanol extract was obtained.

### (I-3) Retama extract

Immersing 50 g of flowers and branches of Retama in ethanol at room temperature for a week and concentrating the resultant extract, 3.0 g of an ethanol extract was obtained.

### (I-4) Canchalagua extract

Immersing 50 g of the whole of canchalagua grass in ethanol at room temperature for a week and concentrating the resultant extract, 5.2 g of an ethanol extract was obtained.

### (I-5) Preparation of Kakkon-derived extract or its purified fraction

*Kakkon* (Puerariae Radix officinal prescription, 1000 g) was extracted with methanol (5 liters) at room temperature to provide a methanol extract (80 g) (which hereafter may be referred to as *Kakkon* extract). This extract was distributed between water-ethyl acetate. To the aqueous phase n-butanol was further added to conduct another solvent distribution. Distilling the respective solvents off from the resulting ethyl acetate phase and n-butanol phase under reduced pressure, ethyl acetate fraction (18 g) and n-butanol fraction (38.5 g) were obtained. Separating and purifying 1 g of said ethyl acetate fraction on normal phase and reversed-phase silica gel columns, lupeol (24 mg) and isobauerenol (8 mg) were obtained.

### (II) Laminin 5 productivity evaluation test and result

### (1) Culture of keratinocytes

Keratinocytes were isolated from the human preputium and cultured in an epidermal cell growth medium (KGM) having a low calcium concentration. To this culture medium were added bovine pituitary extract and EGF. After cultivation in KGM up to the fourth generation, the cells were treated with trypsin and EDTA to suspend adherent cells. Then, the culture was filtered to remove cell aggregates and thereby to obtain a homogeneous cell suspension. The cells were collected by centrifugation and resuspended in DMEM-F12 (2:I)- 0.1% BSA so as to give a cell density of 8 × 10⁴ per ml. Each 0.5 ml of this cell suspension was added to 0.5 ml of the same medium containing a twofold concentration of each test sample. Using a 24-well plate, incubation was carried out at 37°C for 24 hours. After completion of the incubation, the culture supernatant was transferred to an Eppendorf tube and centrifuged at 10,000 rpm for 5 minutes. The supernatant was transferred to a new tube and stored at -20°C till the day for the determination of laminin 5. In order to solubilize laminin 5 in the cells and that bound to the culture plastics, a tris-HCl buffer (pH 7.4) containing various surfactants was added to each well and allowed to stand at -20°C overnight. On the following day, the mixture was ultrasonicated and frozen again. On the following day, the mixture was thawed again and centrifuged at 10,000 rpm for 5 minutes. The supernatant was transferred to a tube and stored at -20°C till the day for the determination of laminin 5.

### (2) Determination of laminin 5 by the sandwich ELISA method

Laminin 5 present in the culture supernatant and the cell layer was determined by the sandwich ELISA method. A monoclonal antibody (BM165) to the laminin α3 chain of laminin 5 was attached to a solid layer of a 96-well ELISA plate. In order to determine laminin 5 in sandwiched manner, a monoclonal antibody (6F12) to the laminin β3 chain was previously biotinized (b-6F12) and used as the other antibody. In this method, only the heterotrimer (α3β3,γ2) which can exhibit its function was measured, and the heretodimer (β3γ2) was not detected. A sample was added to each of the wells in which a 3% gelatin-phosphate buffer solution containing b-6F12 had previously been placed. The final dilution ratio of the sample in the wells was 1/4 for the culture medium and 1/10 for the cell layer.
After the antigen-antibody reaction was carried out at 37°C for 2 hours, the plate was washed, and an avidin HRP (horseradish peroxidase) solution was added thereto and reacted for a period of 30 minutes to 1 hour. After washing, a solution of ABTS serving as a substrate for HRP was added thereto and the absorbance at 405 nm was measured with an ELISA plate reader. A calibration curve was constructed over a range of 0 to 40 ng/ml.

The sum of the laminin 5 liberated into the culture medium and that remaining in the cell layer was calculated. The yield of laminin 5 was expressed by the relative value to that of the sample to which none of the plant extracts was added (control).

### (3) Results

The results are shown in the following Table 1. Also the results of the tests with the fractions derived from the *Kakkon* extracts are shown in Table 2 (the present invention) and Table 3 (comparative example).

**Table 1**

| | Test Sample | Concentration (%) | Laminin 5 yield (%) Relative to Control Not added with Plant Extract |
|---|---|---|---|
| (For comparison) | Centella extract | 0.01 | 56.5 |
| (This invention) | Ononis extract | 0.01 | 151 |
| | Melilot extract | 0.01 | 122 |
| | *Moyashi* extract | 0.01 | 120 |
| | *Adzukl* extract | 0.01 | 174 |
| | *Kakkon* (Puerariae Radix) extract | 0.01 | 245 |
| | Licorice extract (flavonoid fraction) | 0.01 | 141 |
| | Licorice extract (flavonoid fraction) | 0.001 | 118 |
| | Licorice extract | 0.001 | 105 |
| | Blackberry lily extract (Belamcanda chinensis DC.) | 0.001 | 103 |
| | Alstonia scholaris | 0.01 | 239 |
| | Tinospora tuberculata Beumee | 0.01 | 216 |
| | Trigonella foenum-graecum | 0.01 0.0001 | 143 108 |
| | Whey extract | | |
| | Palo Amarillo (Bocconia arborea) extract | 0.01 | 310 |
| | Winged bean (Psophocarpus tetragonolobus) | 0.01 | 272 |
| | extract | | |
| | Retama (Cassia spinecens H.) extract | 0.01 | 173 |
| | Canchalagua extract | 0.01 | 152 |

**Table 2 Activity of Further Processed or Purified Fractions of Kakkon Extract as obtained in I. (I-5) (this invention)**

| Test Sample | Concentration (µM) | Laminin 5 yield (%) Relative to the Control |
|---|---|---|
| Butanol fraction | 10 | 118 |
| | 100 | 182 |
| Ethyl acetate fraction | 1 | 126 |
| | 10 | 209 |
| Lupeol | 0.1 | 122 |
| | 1 | 154 |
| Isobauerenol | 1 | 132 |
| | 10 | 137 |

**Table 3 Activity of Isoflavones Contained in KaHon (Comparative Example)**

| Test Sample | Concentration (µM) | Laminin 5 yield (%) Relative to the Control |
|---|---|---|
| Daidzein ^{*1)} | 1 | 93 |
| | 10 | 99 |
| | 100 | 74 |
| Glucoside ^{*2)} | 1 | 93 |
| | 10 | 95 |
| | 100 | 99 |

| | | |
|---|---|---|
| *1): Purchased from BIOMOL Research Labs. Co. *2): 8-β-D-glucopyranosyl-7-hydroxy-3-(3',4'-dihydroxyphenyl)-4H-1-benzopyran-4-one as described in JP-1992-282305A | | |

### (III) Test for confirming effect of improving skin-aging symptoms using Ononis extract and the result

A consecutive application test (twice a day in the morning and night, four weeks) of the skin cream as formulated in Example 1 given later, was conducted with 22 healthy female volunteer subjects of 40-68 years old (51 years old on the average) who showed notable wrinkle and fine wrinkle formation and deterioration in skin elasticity. At the end of said test, the following skin parameters of the subjects were measured in an atmosphere of constant temperature and constant humidity, and the data were compared with those before the test (at the time using cosmetics not containing the Ononis extract).

### Measured parameters

Replica of the corner of the eye of each subject was taken with silicone replica (Silflo, Flexico Developments) and number and lengths of the lines at the corners were measured.

Water content of stratum corneum was measured with Corneometer (Courage & Khazaka).

Viscoelasticity of the skin was measured with Cutometer (Courage & Khazaka). Following the accepted practice, 2 seconds' suction and 1 second's release cycle was repeated 3 times, and the skin elongation (Uf values) and viscosity/elasticity ratio (Uv/Ue values) in the first and third cycles were compared with those values before the test.

Condition of the skin texture and uniform elimination of stratum corneum of each subject were visually rated by an experts' panel, from the stratum corneum taken with an adhesive tape. The subjects also were required to answer a questionnaire relating to the skin conditions before and after the test.

### Results (comparison with those before the consecutive use)

· Result of the replica analysis (Test: Paired Student t-test)
   Number of wrinkles -8% (p=0.003)
   Length of wrinkles -9% (p=0.010)
   Presence of statistically significant anti-wrinkle effect of the cream of Example 1 was confirmed.
· Water content of the stratum corneum (Test: Paired Student t-test) +22% (p=0.0001)

Presence of statistically significant moisture-retention effect of the cream of Example 1 was confirmed.
Viscoelasticity of the skin (Test: Paired Student t-test)
Uf (maximal amplitude, first cycle) -21% (p=0.0063)
Uf (maximal amplitude, third cycle) -29% (p=0.0007)
Uv/Ue -28% (p=0.002)

It was confirmed that the application of the cream of Example 1 significantly increased the skin elasticity. · Skin texture (Test: Paired Wilcoxon test)
Skin texture condition (Skin Network Sharpness) +23% (p=0.0022)
Uniform removal of the stratum corneum (skin surface aspect) +33% (p=0.0001)

Presence of significant skin texture improving effect of the cream of Example 1 was confirmed. · Answers by the volunteers to the questionnaire

Percentage of the subjects who acknowledged efficacy on the following items

| | |
|---|---|
| skin softeness | 91% |
| wrinkle reduction | 45% |
| fine wrinkle reduction | 77% |
| skin texture improvement | 77% |
| full feeling improvement | 82% |
| elasticity improvement | 73% |
| skin smoothness | 82% |
| skin condition improvement | 95% |
| superior effect to cosmetics used | |
| before the test (cosmetics not | |
| containing the Ononis extract) | 90% |

From the foregoing, skin cream containing Ononis extract as shown in Example 1 is considered to be an excellent anti-aging cream because it has anti-wrinkle effect and effects for improving moisture retention, suppleness, elasticity and texture of the skin. Also safety has been confirmed and nearly all of the volunteers were found feeling that the tested cream was more effective than the cosmetics not containing the Ononis extract which they had been using.

### (IV) Test for evaluating inhibitory effect of winged bean extract on skin wrinkle formation by UVB and the result

Inhibitory effect of a methanol extract of winged bean [see: (I)-(I-2)] on skin wrinkle formation by UVB was evaluated. Three groups of hairless mice (5 mice per group) were organized, avoiding unbalance in body weight and skin conditions among the groups, as follows:

### untreated group (no UV radiation; no medicine application)

UV radiation + solvent (80% ethanol) applied group

UV radiation + 1% (w/w) winged bean's methanol extract (prepared as 80% ethanol solution) applied group.

Wrinkle formation in the mice was conducted by the Schwartz's method (*1) with partial modification, comprising repetitive radiation of UVB onto the mice' backs. The mice (4 weeks old) of the UV radiation groups were given UVB irradiation at their backs in the manner known per se (light source: Toshiba Electric, TOSHIBA FL-20 SE fluorescent lamp) three times a week, for 10 consecutive weeks (*2, *3), accompanied by application of the test substance (5 times a week, 100 µl per application). On the UVB radiation days, the substance was applied after the UV radiation, in order to avoid the influence of UV on the test substance. The UV radiation was conducted after wiping the mice' backs with ethanol, to remove any residual test substance on the skin surface. The radiation dosage in the initial week was 36 mJ/cm²/radiation, which was gradually increased from the second and subsequent weeks, up to 216 mJ/cm²/radiation in the tenth week. The total radiation dosage was 4.6 J/cm².

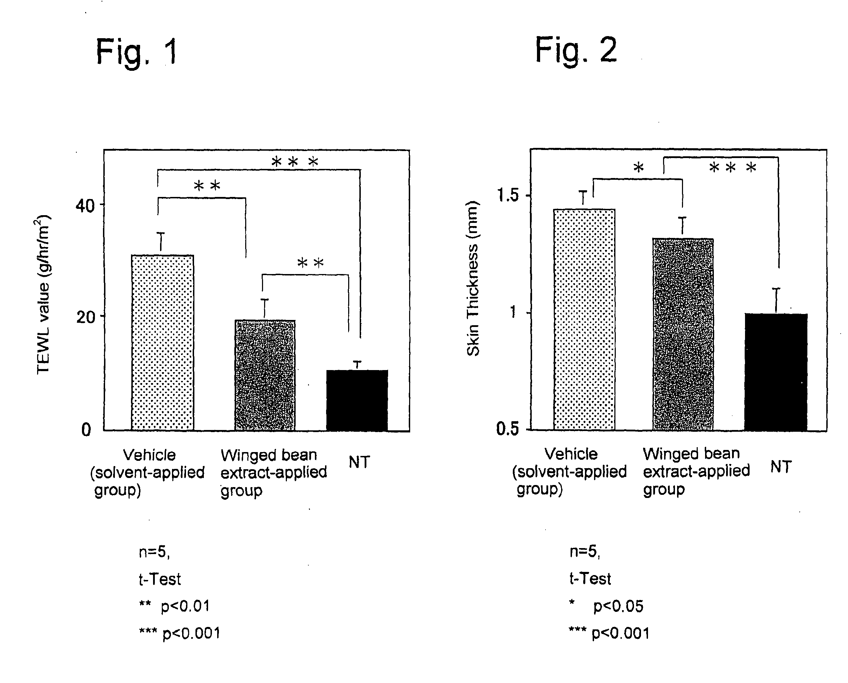

After ten weeks of the initiation of the consecutive UV irradiation, amount of trans-epidermal water loss (TEWL) and skin thickness at the irradiated regions were measured and compared among the test groups by t-test (Figs. 1 and 2). Tewameter (Courage & Khazaka) was used for the TEWL measurement, and Peacock, Dial Thickness Gage (Ozaki Seisakusho), for measuring skin thickening by UV

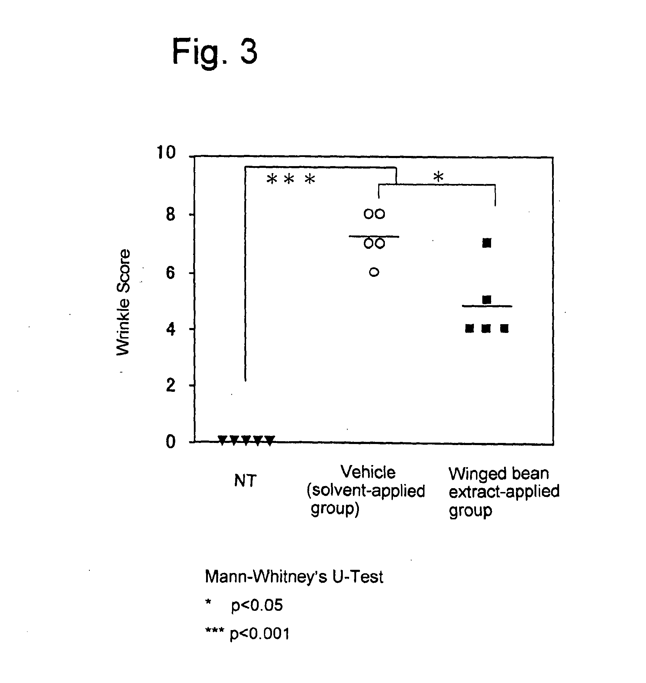

Further, the mice' backs were photographed and extent of wrinkle formation in the mice was converted to wrinkle scores following Bissett's method (*4) with partial modification i.e., without identifying the group to which the mice belonged, according to the rating standard shown in the following Table 4 (Fig. 3). This work was done by three scorers independently of each other, and Man-Whitney's U examination was conducted concerning the groupwise scores.

### Table 4

Table of visual rating standard for wrinkle formation Score: evaluation standards
0: no wrinkle observed
1: shallower, shorter or less number of wrinkles than those of score 2
2: shallow wrinkles observed
3: deeper or longer wrinkles than those of score 2; shallower, shorter or less number of wrinkles than those of score 4
4: shallow wrinkles observed over the entire region
5: deeper or longer wrinkles than those of score 4; shallower or shorter wrinkles than those of score 6
6: deep and long wrinkles observed
7: deep and long wrinkles increased from those of score 6, which are shallower or shorter than those of score 8
8: deep and long wrinkles observed over the entire region

In Fig.1, in contract to the notable TEWL value rise in the solvent (80% ethanol)-applied group (control), in the winged bean extract-applied group such rise was significantly inhibited. This fact clearly indicates the winged bean extract possesses recovery-promoting effect on skin barrier disturbance caused by light.

Fig.2 also clearly illustrates that the skin thickening caused in the control group by UV was significantly inhibited in the winged bean extract-applied group.

Furthermore, as is clear from Fig.3, deep wrinkle formation was observed in the solvent (80% ethanol)-applied group. In contrast, wrinkle formation was markedly inhibited for the group applied with the winged bean extract of the present invention, the effect being confirmed statistically significant.

Based on the foregoing, winged bean extract is confirmed to be effective for preventing or inhibiting skin abnormality caused by ultraviolet rays.

### Literature references

*1 Haratake A, Uchida Y, Schmuth M, Tanno O, Yasuda R, Epstein JH, Elias PM, and Holleran WM: UVB-induced alterations in permeability barrier function: role for epidermal hyperproliferation and thymocyte-mediated response. J. invest. Dermatol. 108 : 769-775, 1997.
*2 Naganumaa M, Yagi E, and Fukuda M: Delayed induction of pigmented spots on UVB-irradiated hairless mice. J. Dermatol. Sci. 25:29-35, 2001.
*3 Schwartz E: Connective tissue alterations in the skin of ultraviolet irradiated hairless mice. J. Invest. Dermatol. 91: 158-161, 1988.
*4 Bissett DL, Hannon DP, and Orr TV: An animal model of solar -aged skin : histological, physical, and visible changes in UV-irradiated hairless mouse skin. Photochemistry and Photobiology 46:367-378, 1987.

### Examples of compositions or medicines for external application to the skin

### Example 1 Cream

| (Recipe) | | |
|---|---|---|
| A. | Cyclomethicone | 10.0 wt% |
| | Cetyl octanoate | 5.0 |
| | Squalane | 10.0 |
| | Meadow-foam oil | 3.0 |
| | Tetrahydrotetramethylcyclotetrasiloxane | 5.0 |
| | Dimethicone polyol | 3.0 |
| | Quatanium-18 hectorite | 2.0 |
| | Perfume | a proper quantity |
| B. | Edetate | 0.1 |
| | Polyethylene glycol 6000 | 1.0 |
| | Glycerine | 5.0 |
| | Dipropylene glycol | 5.0 |
| | Tranexamic acid | 0.5 |
| | Magnesium phosphoascorbate | 0.1 |
| | Sodium hyaluronate | 0.01 |
| | L-arginine hydrochloride | 0.01 |
| | Ononis extract | 0.1 |
| | Bupleurum falcatum L. extract | 0.1 |
| | Methylparaben | 0.2 |
| | Ion-exchange water | balance |

A components were homogeneously dispersed, and to its oil phase part B components as dissolved at room temperature was slowly added under dispersing with a homogenizing mixer.

### Example 2 Wrinkle-preventive cream

| (Recipe) | |
|---|---|
| Stearic acid | 2.0 wt% |
| Stearyl alchol | 7.0 |
| Hydrous lanolin | 2.0 |
| Squalane | 5.0 |
| 2-Octyldodecyl alcohol | 6.0 |
| Polyoxyethylene (25 moles) cetyl alcohol ether | 3.0 |
| Glycerine monostearate | 2.0 |
| Propylene glycol | 5.0 |
| Melilot ethanol extract | 0.05 |
| Turmeric ethanol extract | 0.05 |
| Sodium hydrogensulfite | 0.03 |
| Ethylparaben | 0.3 |
| Perfume | a proper quantity |
| Ion-exchange water | balance |

### (Preparation method)

Propylene glycol was added to ion-exchange water and heated to be maintained at 70°C (aqueous phase). Those other components were mixed, dissolved by heating and maintained at 70°C (oil phase). The oil phase was added to the aqueous phase, subjected to preliminary emulsification, uniformly emulsified with a homogenizing mixer and thereafter cooled to 30°C under thorough stirring.

### Example 3 Cream

| (Recipe) | |
|---|---|
| Solid paraffin | 5.0 wt% |
| Bees wax | 10.0 |
| Vaseline | 15.0 |
| Liquid paraffin | 41.0 |
| Glycerine monostearate | 2.0 |
| Polyoxyethylene (20 moles) sorbitan monolaurate | 2.0 |
| Soap powder | 0.1 |
| Borax | 0.2 |
| *Moyash* 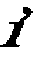 *s* acetone extract | 0.05 |
| Bupleurum falcatum L. ethanol extract | 0.05 |
| Sodium hydrogensulfite | 0.03 |
| Ethylparaben | 0.3 |
| Perfume | a proper quantity |
| Ion-exchange water | balance |

### (Preparation method)

Soap powder and borax were added to ion-exchange water, dissolved by heating and maintained at 70°C (aqueous phase). Other components were mixed, melted by heating and maintained at 70°C (oil phase). The oil phase was gradually added to the aqueous phase under stirring to carry out the reaction. After termination of the reaction, the reaction mixture was uniformly emulsified with a homogenizing mixer, followed by cooling to 30°C under thorough stirring.

### Example 4 Cream

| (Recipe) | |
|---|---|
| (A phase) | |
| Stearic acid | 10.0 wt% |
| Stearyl alcohol | 4.0 |
| Butyl stearate | 8.0 |
| Glycerine monostearate | 2.0 |
| Vitamin E acetate | 0.5 |
| Vitamin A palmitate | 0.1 |
| Macadamia nut oil | 1.0 |
| Antiseptic | a proper quantity |
| Perfume | do. |

| (B phase) | |
|---|---|
| Glycerine | 4.0 |
| 1,2-Pentanediol | 3.0 |
| Potassium hydroxide | 0.4 |
| Magnesium phosphoascorbate | 0.1 |
| L-arginine hydrochloride | 0.01 |
| Trisodium edetate | 0.05 |
| *Adzuki* ethanol extract | 0.1 |
| *Kakkon* 1,3-butylene glycol extract | 0.5 |
| Ion-exchange water | balance |

### (Preparation method)

The oil phase A and aqueous phase B were each heated to 70°C to complete dissolution. The A phase was added to B phase and emulsified with an emulsifier. The emulsion was cooled using a heat exchanger.

### Example 5 Milky lotion

| (Recipe) | |
|---|---|
| Stearic acid | 2.5 wt% |
| Vaseline | 5.0 |
| Liquid paraffin | 10.0 |
| Polyoxyethylene (10 moles) monooleate | 2.0 |
| Polyethylene glycol 1500 | 3.0 |
| Triethanolamine | 1.0 |
| Carboxyvinyl polymer | 0.05 |
| (Carbopole™ 941, B.F. Goodrich Chemical Company) | |
| Licorice ethyl acetate extract | 0.01 |
| Sodium hydrogensulfite | 0.01 |
| Ethylparaben | 0.3 |
| Perfume | a proper quantity |
| Ion-exchange water | balance |

### (Preparation method)

Carboxyvinyl polymer was dissolved in a small amount of ion-exchange water (A phase). To the remaining ion-exchange water, Polyethylene glycol 1500 and triethanolamine were added, dissolved by heating and maintained at 70°C (aqueous phase). All other components were mixed, dissolved by heating and maintained at 70°C (oil phase). The oil phase was added to the aqueous phase, subjected to preliminary emulsification, and to which the A phase was added, followed by homogeneous emulsification with a homogenizing mixer. Thereafter the emulsion was cooled to 30°C under thorough stirring.

### Example 6 Milky lotion

| (Recipe) | |
|---|---|
| (A phase) | |
| Squalane | 5.0 wt% |
| Oleyl oleate | 3.0 |
| Vaseline | 2.0 |
| Sorbitan sesquioleate | 0.8 |
| Polyoxyethylene oleyl ether (2OEO) | 1.2 |
| Evening primrose oil | 0.5 |
| Antiseptic | a proper quantity |
| Perfume | do. |

| (B phase) | |
|---|---|
| 1,3-Butylene glycol | 4.5 |
| Melissa ethanol extract | 1.5 |
| Ethanol | 3.0 |
| Carboxyvinyl polymer | 0.2 |
| Potassium hydroxide | 0.1 |
| L-arginine L-aspergillate | 0.01 |
| Blackberry lily ethanol extract | 1.5 |
| Alstonia scholaris 1,3-butylene glycol extract | 0.01 |
| Erythritol | 0.5 |
| Sodium hexametaphosphate | 0.05 |
| Ion-exchange water | balance |

### (Preparation method)

The oil phase A and aqueous phase B were each heated to 70°C until complete dissolution. The A phase was added to the B phase, and emulsified with an emulsifier. The emulsion was cooled using a heat exchanger.

### Example 7 Jelly

| (Recipe) | |
|---|---|
| 95% Ethyl alcohol | 10.0 wt% |
| Dipropylene glycol | 15.0 |
| Polyoxyethylene (50 moles) oleyl alcohol ether | 2.0 |
| Carboxyvinyl polymer | 1.0 |
| (Carbopole™ 940, B.F. Goodrich Chemical Company) | |
| Sodium hydroxide | 0.15 |
| L-Arginine | 0.1 |
| Tinospora tuberculata Beumee 50% aqueous | |
| ethanol extract | 7.0 |
| Fenugreek 90% aqueous ethanol extract | 0.5 |
| 2-Hydroxy-4-methoxybenzophenone sodium sulfonate | 0.05 |
| Ethylenediaminetetraacetate trisodium dehydrate | 0.05 |
| Methylparaben | 0.2 |
| Perfume | a proper quantity |
| ion-exchange water | balance |

### (Preparation method)

Carbopole 940 was homogeneously dissolved in ion-exchange water. Separately, Tinospora tuberculata Beumee 50% aqueous ethanol extract, fenugreek 90% aqueous ethanol extract, and polyoxyethylene (50 moles) oleyl alcohol ether were dissolved in 95% ethanol, and the formed solution was added to the aqueous phase. Then the other components were added, neutralized with sodium hydroxide and L-arginine and thickened.

### Example 8 Beautifying essence

| (Recipe) | |
|---|---|
| (A phase) | |
| Ethyl alcohol (95%) | 10.0 wt% |
| Polyoxyethylene (20 moles) octyldodecanol | 1.0 |
| Pantothenyl ethyl ether | 0.1 |
| Palo amarillo methanol extract | 1.5 |
| Methylparaben | 0.15 |

| (B phase) | |
|---|---|
| Potassium hydroxide | 0.1 |

| (C phase) | |
|---|---|
| Glycerine | 5.0 |
| dipropylene glycol | 10.0 |
| Sodium hydrogensulfite | 0.03 |
| Carboxyvinyl polymer | 0.2 |
| (Carbopole™ 940, B.F. Goodrich Chemical Company) | |
| Purified water | balance |

### (Preparation method)

The A phase and C phase were separately homogeneously dissolved, and A-phase was added to C phase and solubilized. Then B phase was added and the formulation was filled into containers.

### Example 9 Toilet lotion

| (Recipe) | |
|---|---|
| (A phase) | |
| Ethanol | 5.0 wt% |
| POE oleyl alcohol ether | 2.0 |
| Oleyl alcohol | 0.1 |
| 2-ethylhexyl-P-dimethylaminobenzoate | 0.18 |
| Perfume | a proper quantity |

| (B phase) | |
|---|---|
| 1,3-Butylene glycol | 9.5 |
| Glycerine | 2.0 |
| Sodium pyrrolidonecarboxylate | 0.5 |
| Nicotinic acid amide | 0.3 |
| Winged bean 1,3-butylene glycol extract | 0.1 |
| β-cyclodextrin | 1.0 |
| Erythritol | 0.05 |
| Ion-exchange water | balance |

### (Preparation method)

The alcoholic A phase was added to the aqueous B phase to be solubilized to provide a toilet lotion.

### Example 10 Pack

| (Recipe) | |
|---|---|
| (A phase) | |
| Dipropylene glycol | 5.0 wt% |
| Polyoxyethylene (60 moles) hardened castor oil | 5.0 |

| (B phase) | |
|---|---|
| Retama 100% ethanol extract | 0.01 |
| Canchalagua ethyl acetate extract | 0.1 |
| Olive oil | 5.0 |
| Tocopherol acetate | 0.2 |
| Ethylparaben | 0.2 |
| Perfume | 0.2 |

| (C phase) | |
|---|---|
| Sodium hydrogensulfite | 0.03 |
| Polyvinyl alcohol | |
| (90% saponified; degree of polymerization 2000) | 13.0 |
| Ethanol | 7.0 |
| Purified water | balance |

### (Preparation method)

The A phase, B phase and C phase were each homogeneously dissolved, and the B phase was added to A phase to be solubilized.
Then the mixture was added to C Phase and filled into containers.

### Example 11 Solid foundation

| (Recipe) | |
|---|---|
| Talc | 43.1 wt% |
| Kaolin | 15.0 |
| Sericite | 10.0 |
| Zinc flower | 7.0 |
| Titanium dioxide | 3.8 |
| PMMA spherical powder | 5.0 |
| Yellow iron oxide | 2.9 |
| Red iron oxide | 1.0 |
| Black iron oxide | 0.2 |
| Squalane | 8.0 |
| Isostearic acid | 4.0 |
| POE sorbitan monooleate | 3.0 |
| Isocetyl octanoate | 2.0 |
| Ononis ethanol extract | 0.5 |
| Antiseptic | a proper quantity |
| Perfume | do. |

### (Preparation method)

The powdery components from above talc down to black iron oxide were thoroughly mixed with a blender. To the mixture the oil components from squalane down to isocetyl octanoate, Ononis ethanol extract, antiseptic and perfume were added and together well kneaded. The formed composition was filled in containers and molded.

### Example 12 Emulsified foundation (cream type)

| (Recipe) | |
|---|---|
| (Powder component) | |
| Titanium dioxide | 10.3 wt% |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethylcyclopentasiloxane | 11.5 |
| Liquid paraffin | 4.5 |
| Polyoxyethylene-modified dimethylpolysiloxane | 4.0 |

| (Aqueous phase) | |
|---|---|
| Purified water | 50.0 |
| 1,3-Butylene glycol | 4.5 |
| *Kakkon* 100% ethanol extract | 1.5 |
| Sorbitan sesquioleate | 3.0 |
| Antiseptic | a proper quantity |
| Perfume | do. |

### (Preparation method)

The aqueous phase was heated under stirring, to which thoroughly mixed and pulverized powder component was added and treated with a homogenizing mixer. Further, heated and mixed oil phase was added and treated with the homogenizing mixer. Perfume was added to the homogenous mixture under stirring, followed by cooling to room temperature.

### Field of industrial utilization

As so far explained, the compositions or medicines for external application to the skin following the present invention possess excellent laminin 5 productivity potentiating action, and have the efficacy of ameliorating reduced skin function indicated by wrinkles, sagging and hardening of the skin accompanying structural change in the basement membrane induced by aging or photodegradation, and maintaining elastic, youthful and healthy skin condition. Accordingly, the present invention is useful for cosmetic makers or drug makers which supply such preparations to the public.

## Claims

1. A composition for preventing or ameliorating aging of the skin, which comprises an effective amount of a substance capable of potentiating laminin 5 productivity in the epidermal cells and components other than said substance, which are customarily blended in cosmetics or drug for external application to the skin, said substance being a plant extract, in which the plant is winged bean (botanical name: Psophocarpus tetragonolobus) belonging to Legminosae Psophocarpus.

2. A composition according to Claim 1, in which the extract is obtained with the use of a solvent selected from the group consisting of lower alkanol, lower alkylene glycol, lower alkyl-lower alkylketone and lower alkanoic acid lower alkyl, as the extraction solvent.

3. A composition according to Claim 1, in which the extract is obtained with the use of a solvent selected from the group consisting of alcohols such as methanol, ethanol and the like, hydrous alcohols as well as 1,3-butylene glycol.

4. A composition according to Claim 1, in which the prevention or amelioration of skin aging signifies inhibition of wrinkle formation in the skin or improvement in wrinkled condition.

5. Use of a substance capable of potentiating laminin 5 productivity in the epidermal cells for formulating compositions for preventing or improving aging in the skin, where said substance being a plant extract, in which the plant is winged bean (botanical name: Psophocarpus tetragonolobus) belonging to Legminosae Psophocarpus.

6. The use according to Claim 5, in which the extract is obtained with the use of a solvent selected from the group consisting of lower alkanol, lower alkylene glycol, lower alkyl-lower alkylketone and lower alkanoic acid lower alkyl, as the extraction solvent.

7. The use according to Claim 5, in which the extract is obtained with the use of a solvent selected from the group consisting of alcohols such as methanol, ethanol and the like, hydrous alcohols as well as 1,3-butylene glycol.

8. The use according to any one of Claims 5 - 7, in which the prevention or amelioration of skin aging signifies inhibition of wrinkle formation in the skin or improvement in wrinkled condition.

9. A method for preventing or improving aging in the skin which comprises topical administration of an effective amount of a substance capable of potentiating laminin 5 productivity in the epidermal cells onto the skin of individuals desiring to prevent or improve aging in the skin, in which said substance being a plant extract, in which the plant is winged bean (botanical name: Psophocarpus tetragonolobus) belonging to Legminosae Psophocarpus.

10. A method according to Claim 9, in which the extract is obtained with the use of a solvent selected from the group consisting of lower alkanol, lower alkylene glycol, lower alkyl-lower alkylketone and lower alkanoic acid lower alkyl, as the extraction solvent.

11. The method according to Claim 9, in which the extract is obtained with the use of a solvent selected from the group consisting of alcohols such as methanol, ethanol and the like, hydrous alcohols as well as 1,3-butylene glycol.

12. The method according to Claim 9, in which the prevention or amelioration of skin aging signifies inhibition of wrinkle formation in the skin or improvement in wrinkled condition.
